# EUROPEAN PATENT APPLICATION

(11) **EP 2 486 845 A2**
(43) Date of publication of application: **15.08.2012**
(21) Application number: 12154377.1
(22) Date of filing: 08.02.2012
(51) Int. Cl.: A61B 1/00, A61B 1/12

(54) **Covering apparatus for an endoscope lens**

(30) Priority: 09.02.2011 US 201161440991 P; 30.01.2012 US 201213361194
(71) Applicant: Tyco Healthcare Group LP, Mansfield, MA 02048 (US)
(72) Inventor: O'Prey, Cormac, Bishops Stortford, Hertfordshire CM23 4HH (GB); Scott, Valerie Anne, Cambridge, CB4 2DB (GB)
(74) Representative: Soames, Candida Jane

(57) **Abstract**

A covering apparatus (132) for a surgical viewing instrument. The apparatus includes a tape (135) having a first length overlying the viewing portion (135v) of the surgical viewing instrument, the tape movable across the viewing portion to move the first length away from the viewing portion and advance a second cleaner length of tape to a position overlying the viewing portion.

## Description

### BACKGROUND

This application claims priority from provisional application serial no. 61/440,991, filed February 9, 2011, the entire contents of which are incorporated herein by reference.

### Technical Field

The present disclosure relates to apparatus configured to cover the lens of minimally invasive viewing instrument.

### Background of Related Art

Minimally invasive surgery has become increasingly popular in recent years. Minimally invasive surgery eliminates the need to cut a large incision in a patient, thereby reducing discomfort, recovery time, and many of the deleterious side effects associated with traditional open surgery. Minimally invasive viewing instruments, *e.g.*, laparoscopes and endoscopes, are optic instruments to facilitate the viewing of internal tissues and/or organs.

Laparoscopic surgery involves the placement of a laparoscope in a small incision in the abdominal wall of a patient to view the surgical site. Endoscopic surgery involves the placement of an endoscope in a naturally occurring orifice, *e.g.*, mouth, nose, anus, urethra, and vagina to view the surgical site. Other minimally invasive surgical procedures include video assisted thoracic surgery and cardiovascular surgery conducted through small incisions between the ribs. These procedures also utilize scopes to view the surgical site.

A typical minimally invasive viewing instrument, *e.g.,* a laparoscope or an endoscope includes a housing, an elongated lens shaft extending from one end of the housing, and a lens that is provided in the distal end of the lens shaft. A camera viewfinder extends from the other end of the housing. A camera is connected to the housing and transmits images of the surgical field viewed through the lens to a monitor on which the images are displayed. During a surgical procedure, the distal end portion of the lens shaft is extended into the patient, while the proximal end portion of the lens shaft, the housing and the camera viewfinder remain outside the patient. In this manner, the laparoscope/endoscope is positioned and adjusted to view particular anatomical structures in the surgical field on the monitor.

During insertion of an endoscope or a laparoscope into the body and during the surgical procedure, debris, *e.g.*, organic matter and moisture, may be deposited on the lens of the endoscope. The buildup of debris and condensation on the lens impairs visualization of the surgical site, and often necessitates cleaning of the lens.

### SUMMARY

In one aspect, the present disclosure provides a covering apparatus for a surgical viewing instrument having an elongated shaft including a viewing portion at a distal end thereof The covering apparatus comprises a tape having a first length overlying the viewing portion, the tape movable across the viewing portion to move the first length away from the viewing portion and advance a second cleaner length of tape to a position overlying the viewing portion.

In some embodiments, the apparatus can include a first roller, a second roller, a third length of tape, and a fourth length of tape, the third length of tape wound about the first roller in response to movement of the first length of tape away from the viewing portion, and the fourth length of the tape unwound from the second roller in response to movement of the first length of tape away from the viewing portion. Winding and/or unwinding of the tape about the rollers may be caused through rotation of the rollers. The rotation of the rollers may be restricted in a first direction by a ratchet-type mechanism.

The tape may be transparent to facilitate a high degree of visibility therethrough. In other embodiments, the tape may be alternately transparent and opaque. In still further embodiments, the tape may include cut-out portions. The tape may also include substances and/or materials, *e.g.*, a coating, that facilitate cleaning, *e.g.,* the removal of debris and/or moisture from or near the viewing portion of the viewing instrument.

In another aspect of the present disclosure, a cleaning apparatus is provided for positioning on a surgical viewing instrument having a viewing portion. The cleaning apparatus includes an elongated cleaning member having a first portion to overlie the viewing portion to enable viewing through the viewing portion, a second portion composed of a material to clean the viewing portion and advanceable across the viewing portion to clean the viewing portion, and a third portion advanceable to overlie the viewing portion to enable viewing through the viewing portion. The second portion is positioned between the first and third portions, and the second portion is movable across the viewing portion as the first portion is moved away from the viewing portion and the third portion is movable over the viewing portion as the second portion is subsequently moved away from the viewing portion.

In some embodiments, the first portion provides a shield for the viewing portion. The first portion can be composed of a substantially transparent material. In some embodiments, the first portion and/or the third portion includes a cutout.

In some embodiments, the cleaning apparatus includes a sheath mountable over at least a distal portion of the surgical viewing instrument.

In some embodiments, the cleaning member is supported on first and second opposing sides of the viewing instrument.

In some embodiments, the cleaning apparatus is removably mounted to the surgical viewing instrument.

In another aspect, the present disclosure provides a method for covering a lens of a scope comprising the steps of providing a covering device having first, second and third portions, positioning the first portion over the lens of the scope, moving the first portion away from the lens for placement of the second portion over the lens, and moving the second portion away from the lens for placement of the third portion over the lens.

In some embodiments, the first and third portions can enable viewing through the lens and the second portion can include a cleaning material.

These and other features of the present disclosure will be more fully described with reference to the appended figures.

### BRIEF DESCRIPTION OF THE DRAWINGS

By way of description only, embodiments of the present disclosure will be described herein with reference to the accompanying drawings, in which:

Fig. 1 is a perspective view of a surgical viewing instrument including a lens covering apparatus in accordance with an embodiment of the present disclosure;

Fig. 1A is a perspective view of the distal portion of an alternate covering apparatus of the present disclosure;

Fig. 1B is an enlarged perspective view of the distal portion of the covering apparatus of another alternate embodiment of the present disclosure;

Fig. 1C is a perspective view of the distal portion of yet another alternate embodiment of the covering apparatus of present disclosure;

Fig. 2 is an enlarged perspective view of a portion of the covering apparatus of Figure 1;

Fig. 3 is an enlarged view of the ratchet mechanism of Fig. 2;

Figs. 4 and 5 are perspective views of the covering apparatus of Figure 1 showing movement of the tape across the lens of the viewing instrument;

Fig. 6 is a perspective view of another embodiment of the covering apparatus of the present disclosure;

Fig. 7 is a perspective view of yet another alternate embodiment of the covering apparatus of the present disclosure;

Fig. 8 is an enlarged perspective view of a portion of another embodiment of the covering apparatus of the present disclosure; and

Fig. 9 is an enlarged perspective view of yet another alternate embodiment of the covering apparatus of the present disclosure.

### DETAILED DESCRIPTION

Particular embodiments of the present disclosure will be described with reference to the accompanying drawings. In the figures and in the description that follow, in which like reference numerals identify similar or identical elements, the term "proximal" will refer to the end of the device that is closer to the operator during use, while the term "distal" will refer to the end that is further from the operator during use.

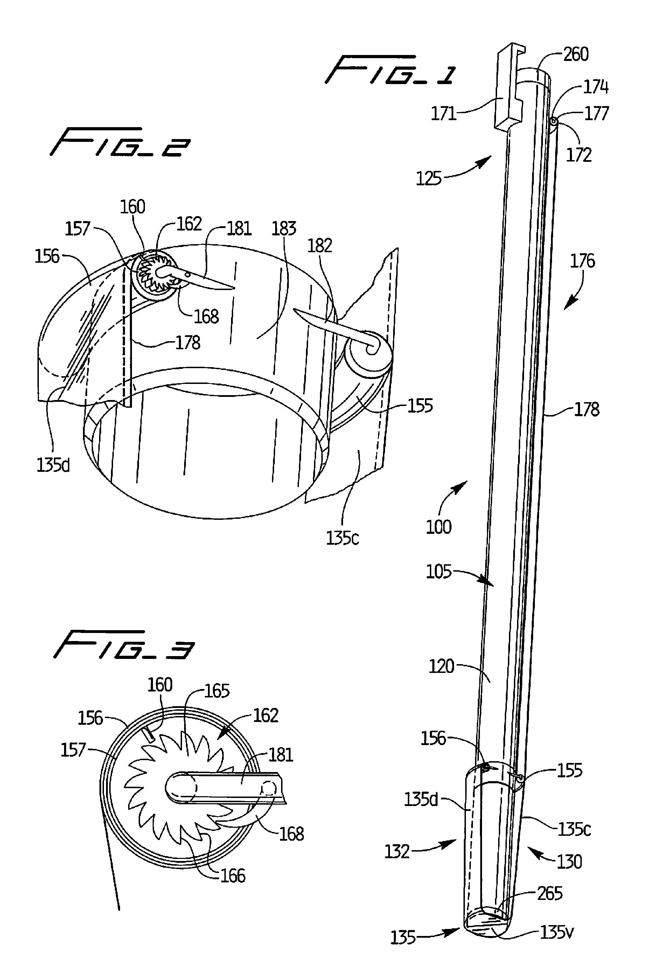

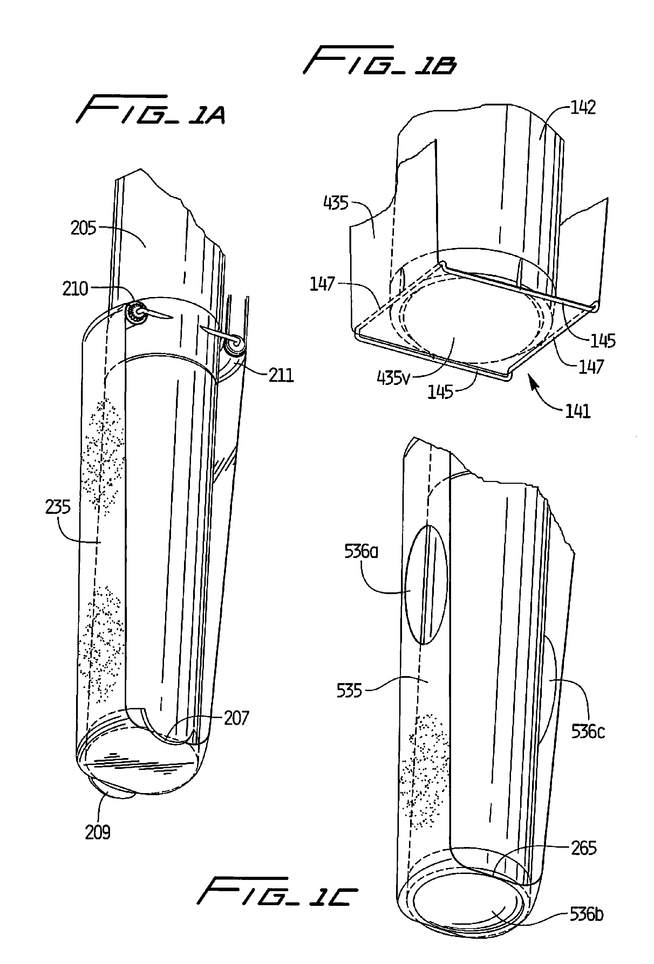

Embodiments of the covering apparatus that are configured and adapted to facilitate maintenance of a substantially unobstructed view through a viewing portion (lens) of a surgical viewing instrument will now be described with reference to Figs. 1-9. The covering apparatus disclosed herein includes a tape or film that is movable across the lens to provide a clearer viewing through the lens of the viewing instrument. The viewing instruments and lens covering apparatus disclosed herein are configured and adapted to be used with minimally invasive surgical instruments and/or during a minimally invasive surgical procedure. The viewing instrument can be in the form of an endoscope (*e.g.* a thoracoscope, laparoscope, etc.). An endoscope typically includes an endoscope housing or body which can be rigid or flexible, depending on its surgical application. A camera viewfinder, *e.g.* an eyepiece, is located at a proximal (imaging) end of the scope housing. A lens is provided at the distal end of the scope body.

In typical use of the endoscope, the viewfinder is adapted to sight images of a surgical field in the patient, *e.g.* an abdominal cavity, thoracic cavity, etc., as the position of the scope is adjusted to view a particular anatomical structure or structures in the surgical field. The camera is adapted to receive images of the surgical field sighted through the lens and transmit the images to a monitor that is connected to the camera and on which the images of the surgical field are displayed. That is, a visual display device is operatively connected to the eyepiece to convert the optical signal into a video signal to produce a video image on the monitor (or for storage on select media). Accordingly, the monitor enables a surgical team to view the anatomical structure or structures in the surgical field inside the patient as the surgical procedure is carried out using minimally invasive or endoscopic surgical instruments. Throughout the surgical procedure, biological tissue or matter has a tendency to contact and build up on the lens of the scope. This tends to obscure the images of the surgical field as they are displayed on the monitor.

The apparatus of the present disclosure enables maintaining a clear view of the scope lens during the surgical procedure to maintain a clear image. This is achieved in some embodiments by covering the lens with a clean portion of tape or film. During insertion and/or during the surgical procedure, if a portion of the tape or film is covered with debris which obstructs viewing, the tape is rolled to advance a cleaner portion of the tape over the lens. The tape or film can continue to be rolled to continuously advance cleaner portions over the scope lens as desired. In alternate embodiments, the tape or film has cutouts which are moved over the lens after the tape is advanced to wipe the lens to provide a clearer viewing through the lens.

Turning first to Figures 1-5, minimally invasive instrument 100 has a covering apparatus 132 coupled thereto. The covering apparatus 132 includes an elongated cylindrical tube or sleeve (sheath) 105, which includes a proximal end 125, a distal end 130, and an outer surface 120. A viewing instrument 260 is receivable within the sheath 105. It should be appreciated that although sleeve 105 in Fig. 1 extends the length of the viewing instrument 260, the sleeve alternatively can extend only over a distal portion of the viewing instrument 260. The viewing instrument 260 includes a viewing portion or lens 265 at a distal end. During the course of a surgical procedure, the lens 265 is susceptible to obstruction by debris and/or moisture which may distort and/or obstruct viewing through the lens 265.

To facilitate maintenance of an unobstructed viewing portion 265, covering apparatus 132 is operably coupled to the tube or sleeve (sheath) 105. It should be appreciated that in alternate embodiments, the covering apparatus can be coupled directly to the outer surface of the viewing instrument 260 without the need for a tube or sheath.

As seen in Fig. 1, the covering apparatus 132 includes a length of tape or film 135 5 that extends along both sides of the surgical viewing instrument and over the viewing portion 265 thereof The length of tape 135 begins at a first tube 177 at a proximal end of the sleeve 105 and extends along the length of the sleeve 105, over guide tube 155 which spaces the tape 135 from sleeve 105, around the viewing portion 265 and proximally to second tube 156. During the course of a surgical procedure, a clean portion of the tape 135 is advanced from the first tube 177 and a dirty portion of the tape 135 is advanced and received within a second tube 156 after it passes by lens 265. For example, as shown in Figure 1, tape portion 135v overlies the lens 265, a clean portion 135c is on one side of the sleeve 105 and instrument 260 and portion 135d is on the other side. Portion 135v can be moved away from the lens 265 and portion 135d will be taken up within tube 156.

A roller 172 is positioned within tube 177 and a roller 157 is positioned within tube 156. A length of tape is wound about each roller. In this manner, as the roller 157 within tube 156 is rotated, the tape 135 is advanced around the viewing portion 265 as it is unwound from roller 172 and wound about roller 157. Alternately, the roller 172 in tube 177 can be actuated to move the tape 135 in the other direction, i.e. to wind about the roller 172 in tube 177 and unwind from the roller 157 in tube 156. In other words, in this alternate embodiment, the roller 172 instead of roller 157 is the take up roller and the roller 157 instead of roller 172 is the supply roller.

A viewing portion 135v of the tape 135 in Fig. 1 is shown in a position overlying the viewing portion (lens) 265 of the viewing instrument 260. In some embodiments, the tape portion 135v can be in abutting relationship with the viewing portion 265. During use, when the viewing portion 135v is covered with too much debris so as to obstruct the viewing capability of the surgical viewing instrument 260, the roller 157 within tube 156 can be rotated (*e.g.* clockwise) to move portion 135v past the lens 265 and toward the roller 157 to position a clean portion 135c of the tape 135 over the lens 265. Such advancement of tape 135 can be performed during the procedure to continue removing dirty portions of the tape 135 from the viewing portion 265 and replacing it with cleaner portions of the tape. The dirty portions of the tape moved past the viewing portion 265 are wound about roller 157. Various methods can be used to wind roller 157. In one version, a wire or cable extends from the roller 157 and is actuated by a rotating lever to rotate the roller 157. In another version, by way of example, a pulley system is provided, such as pulley system 176.

Pulley system 176 is provided to rotate the roller 157 within tube 156 to advance the tape 135 from the proximal supply roller 172 within tube 177. The pulley system 176 includes a wire or cable 178 that is connected to roller 157 and wraps around the covering apparatus 132 up to actuator or handle 171. The cable 178, which can be wrapped around roller 157, can be embedded within the tape. A gear can be operably coupled to the cable 178 that is operably coupled to the handle 171. Consequently, as handle 171 is rotated, the cable 178 is pulled proximally to rotate roller 157 clockwise to wind the tape 135 into tube 156 and unwind the tape 135 from within tube 177 as it passes across the viewing portion 265 of the viewing instrument 260. Other mechanisms for rotating the roller 157 within tube 155 are also contemplated.

Alternately, as noted above, the roller 172 within tube 177 can be used to advance the tape 135 in a reverse direction than that described above. In this embodiment, handle 171 can be operably connected to the roller 172 within proximal tube 177 such that rotation of the handle 171 rotates the roller 172 to advance the tape from within tube 156 (unrolled from roller 157) and wind it about the roller 172 in tube 177. In this version, the portion 135d of tape 135 would be moved across the lens 265 after portion 135v is removed.

As can be appreciated, each roller 157, 172 includes the length of tape 135 wound thereabout to form a spool of tape. Tube 156 includes a slot 160 through which one end of the tape 135 extends into to engage the roller 157; tube 177 includes a similar slot 174 through which the other end of the tape 135 extends. Support arms 181, 182 extend from roller 157 and from guide 155, respectively, and are fixedly positioned therein to frictionally engage a band or collar 183 which is frictionally secured over the outer surface of the sleeve 105 to thereby secure the tubes 156 and 155 (and roller 157). The arm 181 can alternatively be coupled to the tube 156 to frictionally secure the tube to the band 183. A similar arm (not shown) can be used to frictionally secure tube 177 and roller 172.

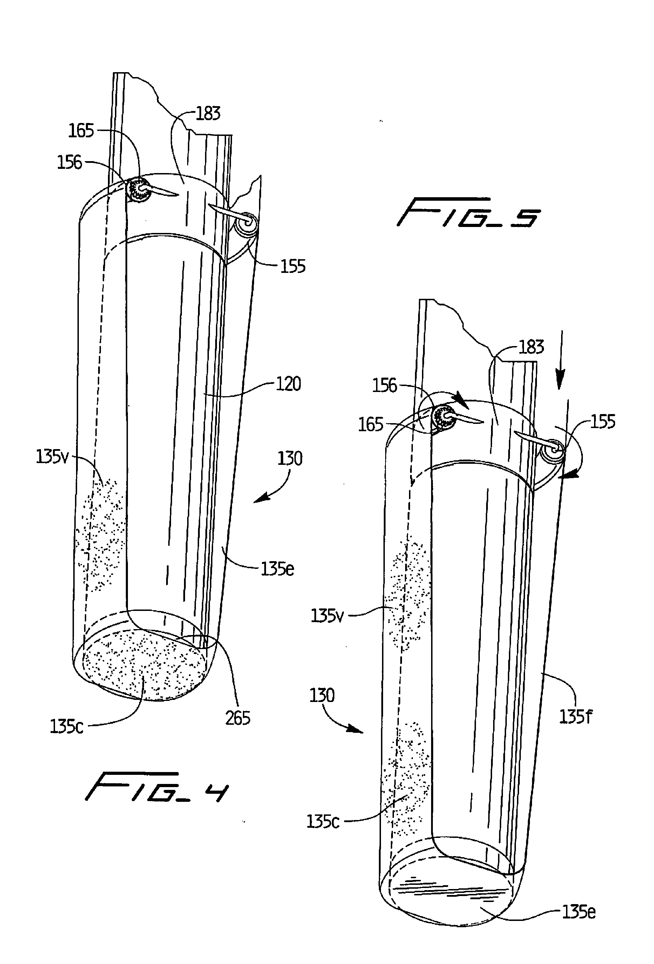

In use, roller 157 is rotated clockwise to translate the tape 135 from roller 172 to remove a dirty portion of the tape 135 from the lens. In Figure 1, clean tape portion 135c is shown along one side of the viewing instrument 260 and portion 135d of tape 135 is on the other side. In Figures 4 and 5 the rollers 157, 172 have already been rotated to remove the dirty portion of the tape 135. More specifically, Figure 4 shows portion 135v translated proximally past the lens 265 with a different viewing portion 135c positioned over the viewing portion 265 of the viewing instrument 260. (Portion 135d has been wound about roller 157 and is now within tube 156). A clean portion of tape 135 is designated by reference numeral 135e. When portion 135c becomes covered with too much debris so as to obstruct viewing of the surgical site, roller 157 is again rotated to advance the tape 135 so that viewing portion 135c is removed from the lens 260 and a new clean viewing portion 135e is positioned over the lens 265 as shown in Figure 5. A clean portion 135f awaits advancement over the viewing portion 265 should portion 135e be covered with too much debris. As can be appreciated, the rollers 157, 172 can be rotated as desired to continue to advance clean portions of the tape 135 over the viewing portion 265 of viewing instrument 260. As noted above, in alternate embodiments, roller 172 can be rotated to wind the tape 135 about roller 172, thereby moving the tape 135 in the opposite direction from that shown in Figures 4 and 5.

Note that a ratcheting mechanism can be provided as shown. As noted above, advancement of the tape 135 is achieved by rotating at least one of the rollers 157, 172, which in turn effects rotation of the other roller and the translation of a portion of the tape 135. To inhibit translation of the tape in the wrong direction, i.e., in a direction that would position a dirty portion of the tape 135 5 over the viewing portion 265, a ratchet mechanism 162 may be operably coupled to one of the rollers, *e.g.*, roller 157. The ratchet mechanism 162, as shown in Figs. 2 and 3, includes a ratchet wheel 165 including teeth 166 and a pawl 168. The ratchet wheel 165 is connected to the roller 157. The pawl 168 is adapted to fit between the teeth 166 to permit rotation of the roller 157 in a first direction and to inhibit rotation of the roller 157 in a second direction. The pawl 168 is in a hinged relationship with arm 181 that is secured to roller 157. It should be appreciated, in alternate embodiments, the ratchet mechanism can be operably coupled to the roller 172.

The positioning of the tape 135 adjacent the viewing portion 265 may form a substantially sealed relationship between the viewing portion 265 and the tape 135. A sealed relationship between the viewing portion 265 and the tape 135 inhibits the depositing and the accumulation of contaminants, *e.g.*, debris and/or moisture, on the viewing portion 265 of the viewing instrument 260.

In the embodiments of Figures 1A and 1B, maintaining positioning of the tape with respect to the viewing portion 265 is facilitated. In Figure 1A, cylindrical tube or sleeve (sheath) 205 has a pair of curved distal extensions 207, 209 forming guides for the tape 235. As tape 235 passes over the lens 265, it is guided between the extensions 207, 209, i.e., it is confined between these extensions. In all other respects, the covering apparatus of Figure 1A is identical to the apparatus of Figure 1, *e.g.*, it contains a roller within tube 210, a guide 211 and a proximal roller within a proximal tube, all identical to respective rollers 157, 172 within tubes 156, 177 and guide 155.

In Figure 1B, an alternate embodiment of a guide is illustrated. More specifically, the guide is in the form of a frame 141 which includes bars 145 that are coupled to the cylindrical tube 142 and overly the surface of the viewing portion 265. These bars 145 help guide the tape 135 therethrough and across the viewing portion 265. Moreover, to smooth the tape 435 and to inhibit optical distortion of the tape 435 by bending of the tape 435, the frame 141 may also include a pair of transverse members or bars 147. As the tape 435 passes over the transverse members 147, irregularities, *e.g.*, bending, that might be present in the tape 135 are smoothed. The pair of members 145 and 147 form a frame 141 that frames the viewing portion 435v of the tape 435.

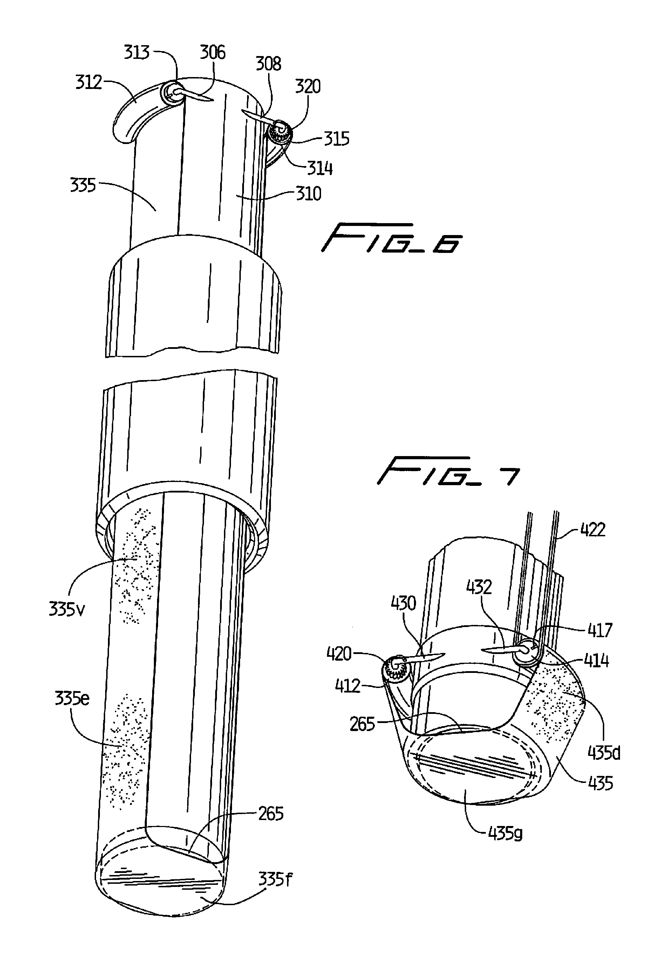

In the alternate embodiment of Figure 6, the tape 335 provides a tighter fit as the tape 335 is wound adjacent the outer surface of the cylindrical tube or sleeve (sheath) 310 positioned over the viewing instrument 260. More specifically, in this embodiment, tube 312 houses a roller 313 and tube 314 houses a roller 315. Both tubes 312, 314 are positioned at the proximal end of the apparatus. Support arms 306, 308 mount the tubes 312, 314 to the outer surface of the sleeve 310 (or directly to the outer surface of the viewing instrument 260 if a sleeve is not utilized) in the same manner as arms 181, 182 of Figure 2. As roller 313 is rotated, tape 335 is wound about the roller 313 within tube 312, and unwound from the roller 315 within tube 314. (Alternatively, roller 313 can form the supply roller and roller 315 can form the take up roller). A ratchet mechanism 320 similar to the ratchet mechanism 162 of Figures 2 and 3 can be provided to limit roller rotation in one direction. The ratchet 320 is provided to operatively engage roller 315 but can alternately operatively engage roller 313 within tube 312 to limit rolling, and therefore movement of the tape 335, in one direction. Since the tape 335 winds around the inner side of the tubes and rollers, the tape 335 can be fit tighter around the viewing instrument and therefore tighter around the viewing portion 265. Note in the illustration of Figure 6, two dirty portions 335v and 335e of tape 335 have already been moved from the viewing portion 265 to enable a clean portion 335f to be placed over the viewing portion (lens) 265.

Note that the tubes and rollers can alternately be positioned on a more distal region of the viewing instrument as shown in Figure 7. In this embodiment, tubes 412, 414 each have a roller rotatably mounted therein similar to rollers 157, 172 of Figure 1. A ratchet mechanism 420 similar to ratchet mechanism 162 can be provided to limit rotation in one direction. Note in Figure 7 the roller within tube 414 forms the take up roller and the roller within tube 412 forms the supply roller as the cable 422 of the pulley system, actuated at the proximal end such as by a handle, rotates the roller within tube 414 to wind up the tape 435 as it extends into slot 417. It is also contemplated that alternatively, the roller within tube 414 could be the supply roller and the roller within tube 412 the take up roller. Arms 430, 432 support the tubes 412, 414 and rollers in the same manner as arms 181, 182 of Figure 2. Note that a dirty portion 435d has been moved away from the viewing portion 265 and a cleaner portion 435g of the tape 435 overlies the viewing portion 265.

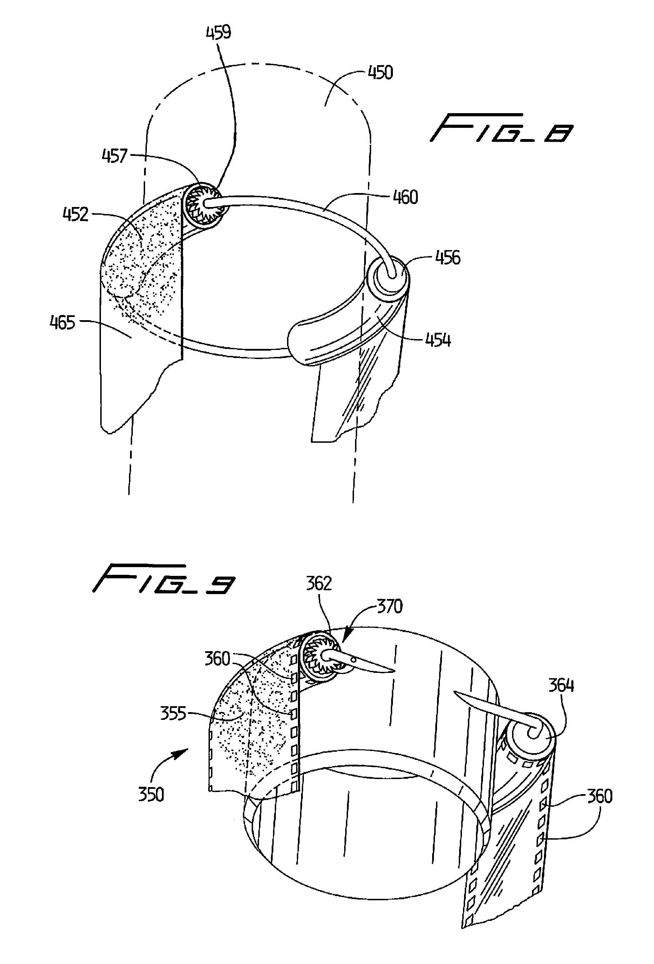

In the embodiment of Figure 8, tubes 452, 454 are connected by a ring 460 which encircles and frictionally engages the cylindrical tube or sleeve (sheath) 450, or encircles to frictionally engage the viewing instrument if the ring 460 is mounted directly to the viewing instrument in embodiments not utilizing a sleeve. Ring 460 extends through the roller 456 which is rotatably positioned within tube 454 and through the ratchet wheel 457 of ratchet 459 and the roller rotatably mounted within tube 452. Ratchet 459 limits movement of the rollers, and thus the tape 465, to movement in one direction as in the aforedescribed ratchets. The rollers can be rotated to advance the tape in the same manner as the rollers described above.

Although the positioning of the tape 135 (and other tapes/films described herein) against the surface of the viewing portion 265 generally inhibits the depositing of debris and/or moisture on the surface of the viewing portion, debris and/or moisture may nonetheless cover the viewing portion and therefore it would be desirable to provide a covering apparatus in the form of a cleaning apparatus having a tape 135 that will clean the surface of the viewing portion 265. Accordingly, for such cleaning, a portion or an entire length of the tape may be impregnated with various materials and/or substances to facilitate cleaning of the viewing portion 265. These materials and/or substances may alter the opacity of the tape. Preferably, the portion of the tape 135 abutting the viewing portion 265 will facilitate an unaltered and unobstructed view of the surgical site. Accordingly, the tape 135 may be impregnated with, or otherwise include, cleaning materials and/or substances in an alternating pattern to facilitate cleaning while still permitting advancing the tape 135 to have a substantially clean transparent portion of the tape 135 abutting the viewing portion 265. The cleaning materials and/or substances may include materials and/or substances that absorb moisture and/or condensation.

In some embodiments, such as shown in Fig. 1C, the tape 535 may include multiple cut-out portions such as cutout portions 536a, 536b and 536b. Tape 535 is advanced to clean the viewing portion 265 of the viewing instrument as the solid (non-cutout) portions of tape 535 move across and in contact with the viewing portion 265, and can contain cleaning materials or substances as described above. The tape 535 is advanced until a second cutout portion, *e.g.*, cutout 536b, is positioned over the viewing portion 265 to provide an unobstructed view. If the viewing portion 265 becomes covered with debris, the tape 535 can be further advanced in the manner described above with respect to the other tapes, *e.g.*, with the use of rotatable rollers, to have a solid portion wipe the lens and position the cut-out portion 536c of the tape 535 over the viewing portion 265. Additional cutouts can be provided to provide for subsequent advancement of the tape.

In an alternate embodiment, the covering apparatus may be coupled to the outer surface of the cylindrical tube or sleeve (sheath) by a C-shaped clip which frictionally engages the sleeve or viewing instrument if mounted directly to the viewing instrument. The clip is biased to apply a compressive force about the sleeve to secure the rollers to the sleeve. In other embodiments not utilizing a sleeve, the C-shaped clip may secure the rollers directly to the viewing instrument 260. In both embodiments, the covering apparatus may be permanently or removably coupled to either the sleeve or to the viewing instrument. In addition, the covering apparatus may be operably and removably coupled to existing surgical instruments.

In another embodiment, a covering apparatus 350 (Fig. 9) includes a tape in the form of a film 355, having transparent sections or formed entirely of a transparent material. The film includes holes 360 along its length. A flexible wire can be provided to interweave through the holes 360 along the length of the film 355. The holes 360 and flexible wire can also be an alternative way to hold and advance the tape 135 of Figure 1. A roller 362 and 364 for each end of the film 355 can include an opening to hold and engage each end of the flexible wire. A ratchet mechanism 370, similar to ratchet mechanism 162, is provided on one of the rollers, *e.g.*, roller 362, to limit rotation of the roller and thereby movement of the film 355, in one direction as the film 355 is advanced over the lens 265. A handle and pulley system, or other actuator, is operatively connected to one of the rollers 362, 364 in the same manner as described above, to unwind the film 355 from one of the rollers, *e.g.,* roller 364, and wind the film 355 about the other roller, *e.g.,* roller 362. A clean and discrete section of the transparent film 355 advances over the lens as the rollers rotate. To this end, the dirty section of the film 355 is replaced with a clean section of the film 355. Alternately, patterns for viewing and cleaning, as in the tape described above, can be provided on film 205.

In alternate embodiments, instead of driving the dirty take up roller at the end of the shaft by the pulley mechanism, the dirty take up roller can be positioned closer to the user's hand so it can be moved, i.e., wound, directly by the user. This simplifies the device. A power supply can also be utilized in alternate embodiments to rotate the roller to advance the tape.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A covering apparatus for a surgical viewing instrument having an elongated shaft including a viewing portion at a distal end thereof, the covering apparatus comprising a tape having a first length overlying the viewing portion, the tape movable across the viewing portion to move the first length away from the viewing portion and advance a second cleaner length of tape to a position overlying the viewing portion.
2. The apparatus of paragraph 1, further comprising a first rotatable roller, a second rotatable roller, a third length of tape, and a fourth length of tape, the third length of tape wound about the first roller in response to movement of the first length of tape away from the viewing portion, and the fourth length of the tape unwound from the second roller in response to movement of the first length of tape away from the viewing portion.
3. The apparatus of paragraph 2, wherein the tape includes first and second ends, the first roller includes a first slot for receiving the first end and the second roller includes a second slot for receiving the second end.
4. The apparatus of paragraph 2, wherein the first and second rollers are mounted to a band positioned over the elongated shaft.
5. The apparatus of paragraph 2, wherein rotation of at least one of the first and second rollers is restricted in one direction.
6. The apparatus of paragraph 5, wherein rotation is restricted in one direction by a ratchet, the ratchet operably engaging at least one of the first and second rollers to restrict the rotation of at least one of the first and second rollers.
7. The apparatus of paragraph 1, wherein the tape has first and second ends supported on opposing sides of the surgical instrument.
8. The apparatus of paragraph 2, wherein the tape further includes at least one flexible cable operably coupled to the first and second rollers.
9. The apparatus of paragraph 1, wherein at least the first length of tape is formed from a transparent material.
10. A cleaning apparatus for positioning on a surgical viewing instrument having a viewing portion, the cleaning apparatus comprising an elongated cleaning member having a first portion to overlie the viewing portion to enable viewing through the viewing portion, a second portion composed of a material to clean the viewing portion and advanceable across the viewing portion to clean the viewing portion, and a third portion advanceable to overlie the viewing portion to enable viewing through the viewing portion, the second portion being positioned between the first and third portions, and the second portion being movable across the viewing portion as the first portion is moved away from the viewing portion and the third portion being movable over the viewing portion as the second portion is subsequently moved away from the viewing portion.
11. The cleaning apparatus of paragraph 10, wherein the first portion provides a shield for the viewing portion.
12. The cleaning apparatus of paragraph 10, wherein the first portion is composed of a substantially transparent material.
13. The cleaning apparatus of paragraph 10, wherein the first portion includes a cutout.
14. The cleaning apparatus of paragraph 10, wherein the cleaning apparatus includes a sheath, the sheath mountable over at least a distal portion of the surgical viewing instrument.
15. The cleaning apparatus of paragraph 13, wherein the third portion includes a cutout.
16. The cleaning apparatus of paragraph 10, further comprising a rotating mechanism for moving the cleaning member across the viewing portion, the cleaning member supported on first and second opposing sides of the surgical viewing instrument.
17. The cleaning apparatus of paragraph 10, wherein the cleaning apparatus is removably mounted to the surgical viewing instrument.
18. A method for covering a lens of a scope comprising;
   providing a covering device having first, second and third portions;
   positioning the first portion over the lens of the scope;
   moving the first portion away from the lens for placement of the second portion over the lens; and
   subsequently moving the second portion away from the lens for placement of the third portion over the lens.
19. The cleaning apparatus of paragraph 18, wherein the first and third portions enable viewing through the lens.
20. The cleaning apparatus of paragraph 18, wherein the second portion includes a cleaning material to clean the lens as it passes thereover.

## Claims

1. A covering apparatus for a surgical viewing instrument having an elongated shaft including a viewing portion at a distal end thereof, the covering apparatus comprising a tape having a first length overlying the viewing portion, the tape movable across the viewing portion to move the first length away from the viewing portion and advance a second cleaner length of tape to a position overlying the viewing portion.

2. The apparatus of claim 1, further comprising a first rotatable roller, a second rotatable roller, a third length of tape, and a fourth length of tape, the third length of tape wound about the first roller in response to movement of the first length of tape away from the viewing portion, and the fourth length of the tape unwound from the second roller in response to movement of the first length of tape away from the viewing portion.

3. The apparatus of claim 2, wherein the tape includes first and second ends, the first roller includes a first slot for receiving the first end and the second roller includes a second slot for receiving the second end.

4. The apparatus of claims 2 or 3, wherein the first and second rollers are mounted to a band positioned over the elongated shaft.

5. The apparatus of claims 2, 3 or 4, wherein rotation of at least one of the first and second rollers is restricted in one direction.

6. The apparatus of claim 5, wherein rotation is restricted in one direction by a ratchet, the ratchet operably engaging at least one of the first and second rollers to restrict the rotation of at least one of the first and second rollers.

7. The apparatus of any preceding claim, wherein the tape has first and second ends supported on opposing sides of the surgical instrument.

8. The apparatus of any of claims 2-7, wherein the tape further includes at least one flexible cable operably coupled to the first and second rollers.

9. The apparatus of any preceding claim, wherein at least the first length of tape is formed from a transparent material.

10. The apparatus of any preceding claim, wherein tape includes a third length composed of a material to clean the viewing portion and advanceable across the viewing portion to clean the viewing portion, the third length positioned between the first and second lengths.

11. The apparatus of claim 10, wherein the first length includes a cutout.

12. The apparatus of any preceding claim, wherein the apparatus includes a sheath, the sheath mountable over at least a distal portion of the surgical viewing instrument.

13. The apparatus of claim 11, wherein the second length includes a cutout.

14. The apparatus of any of claims 1, 7, 10-13, further comprising a rotating mechanism for moving the tape across the viewing portion of the surgical viewing instrument.

15. The apparatus of any preceding claim, wherein the covering apparatus is removably mounted to the surgical viewing instrument.
